# EUROPÄISCHE PATENTSCHRIFT

(11) **EP 0 722 500 B1**
(45) Veröffentlichungstag und Bekanntmachung des Hinweises auf die Patenterteilung: **07.01.2004**
(21) Anmeldenummer: 94929520.8
(22) Anmeldetag: 07.10.1994
(51) Int. Cl.: C12N 15/52, C12P 13/00, C12N 15/74, C12N 1/21

(54) **GENE FÜR DEN BUTYROBETAIN/CROTONOBETAIN-L-CARNITIN-STOFFWECHSEL UND IHRE VERWENDUNG ZUR MIKROBIOLOGISCHEN HERSTELLUNG VON L-CARNITIN**
GENES FOR BUTYROBETAINE/CROTONOBETAINE-L-CARNITINE METABOLISM AND THEIR USE FOR THE MICROBIOLOGICAL PRODUCTION OF L-CARINE
GENES PERMETTANT LE METABOLISME DE LA BUTYROBETAINE/CROTONOBETAINE-L-CARNITINE ET LEUR UTILISATION POUR LA PRODUCTION MICROBIOLOGIQUE DE L-CARNITINE

(30) Priorität: 08.10.1993 CH 303693; 06.01.1994 CH 3694
(43) Veröffentlichungstag der Anmeldung: 24.07.1996
(73) Patentinhaber: LONZA AG, CH-3945 Gampel/Wallis (CH)
(72) Erfinder: ZIMMERMANN, Thomas, CH-3904 Naters (CH); WERLEN, Josef, CH-3916 Ferden (CH)
(74) Vertreter: Weinhold, Peter, Dr.rer.nat. Dipl.-Chem.
(86) Internationale Anmeldenummer: PCT/EP1994/003317
(87) Internationale Veröffentlichungsnummer: WO 1995/010613

(56) Entgegenhaltungen:
- EP-A- 0 122 794
- EP-A- 0 410 430
- EP-A- 0 457 735
- EP-A- 0 543 344

## Beschreibung

Die vorliegende Erfindung betrifft rekombinantes genetisches Material zur Expression der Gene des Butyrobetain/Crotonobetain-L-Carnitin-Stoffwechselweges, Mikroorganismen, die dieses rekombinante genetische Material enthalten, und die Verwendung solcher Mikroorganismen in einem biotechnologischen Verfahren zur Herstellung von L-Carnitin.

L-Carnitin ist eine natürliche, vitaminartige Substanz von großer Bedeutung im menschlichen Stoffwechsel. Essentiell ist L-Carnitin bei der Verwertung von Fettsäuren als Transmittersubstanz der mitochondrialen Membran und somit als Transporteur metabolischer Energie. Wird L-Carnitin in ungenügender Menge vom organismus synthetisiert, muß es zur Vermeidung von Mangelerscheinungen notwendigerweise mit der Nahrung zugeführt werden. Speziell bei der Ernährung von Kleinkindern, die noch nicht zur eigenen L-Carnitin-Biosynthese in der Lage sind, stellt L-Carnitin einen essentiellen Nährstoff dar.

L-Carnitin-Präparate werden als aktive Bestandteile pharmazeutischer Produkte eingesetzt. Angezeigt ist die Supplementierung mit L-Carnitin bei Carnitinmangel und weiteren therapeutischen Indikationen, speziell bei Herzerkrankungen, etc.

Die EP-A-0 543 344 offenbart ein DNA-Fragment mit einer genetischen Sequenz, die für die Verwertung von Betainen codiert, sowie rekombinante Mikroorganismen, die dieses Fragment enthalten.

Die Biosynthese von L-Carnitin in höheren Organismen ist bekannt, weitere Funktionen im und Bedeutung für den Stoffwechsel sind objekte intensiver Forschungsarbeit. Neben einem Stoffwechselweg, welcher für Mikroorganismen speziell der Gattung Pseudomonas beschrieben wird (γ-Butyrobetain-Hydroxylase-Katalyse, Lindstedt et al., Biochemistry 16, 2181-2188, 1977), wird L-Carnitin als Stoffwechselintermediat von gewissen Mikroorganismen, z.B. von Agrobacterium/Rhizobium sp. gebildet.

Aus der EP-A-0 158 194 ist ein Verfahren zur mikrobiologischen Herstellung von L-Carnitin, ausgehend von z.B. γ-Butyrobetain bekannt, bei welchem über traditionelle mikrobiologische Selektionsverfahren eine L-Carnityl-Dehydrogenase negative Produktionsmutante erhalten wird, mit der innerhalb einer Umsetzungszeit von 20 bis 30 Stunden schon relativ gute Ausbeuten an L-Carnitin erhalten werden. Eine weitere Optimierung dieses Verfahrens bezüglich der Volumen-/Zeit-Ausbeuten ist aber mit dieser klassischen mikrobiologischen Methode nicht möglich.

Aufgabe der vorliegenden Erfindung ist es daher, ein wirtschaftlicheres biotechnologisches Verfahren zur Herstellung von L-Carnitin bereitzustellen, wobei L-Carnitin in wesentlich kürzerer Umsetzungszeit mit noch besseren Ausbeuten erhalten wird.

Untersuchungen zum γ-Butyrobetain/Crotonobetain-Stoffwechsel führten zur Identifizierung von fünf Genen, bcoA/B, bcoC, bcoD, bcoE und bcoT, die für Enzyme des γ-Butyrobetain/Crotonobetain-Stoffwechselweges codieren und i.a. in einem Operon, dem sog. Butyrobetain-L-Carnitin-Operon (bco), enthalten sind. Hierbei bedeuten
- bcoA/B:: ein γ-Butyrobetain-CoA-Synthetase(bcoA)/Crotonobetain-CoA-Synthetase(bcoB)-Gen, d.h. ein einziges Gen, das für ein Enzymprodukt codiert, das sowohl γ-Butyrobetain-CoA-Synthetase-Aktivität als auch Crotonobetain-CoA-Synthetase-Aktivität besitzt.
- bcoC:: ein γ-Butyrobetain-CoA-Dehydrogenase-Gen
- bcoD:: ein Crotonobetain-CoA-Hydrolase-Gen
- bcoE:: ein L-Carnityl-Dehydrogenase-Gen; und
- bcoT:: ein potentielles Gen des Transportsystems.

Es wurde gefunden, daß die Genprodukte der Gene bcoA/B, bcoC und bcoD für die Biosynthese des L-Carnitins verantwortlich sind, während bcoE eine Carnitin-Dehydrogenase codiert, die den Abbau des Stoffwechselintermediats L-Carnityl-CoA zu Betain bewirkt. Bei bcoT handelt es sich vermutlich um ein Gen, das für ein Transportprotein eines dem Butyrobetain-Stoffwechsel zugeordneten Transportsystems codiert. Dieses Gen ist für die L-Carnitin-Biosynthese nicht essentiell.

Gegenstand der vorliegenden Erfindung sind demnach DNA-Fragmente und Vektoren, die eines oder mehrere der im γ-Butyrobetain/ Crotonobetain-Stoffwechsel für die Enzyme der Biosynthese von L-Carnitin codierenden Gene bcoC, bcoA/B und bcoD und gegebenenfalls zusätzlich das potentielle Transportgen bcoT umfassen.

Insbesondere ist Gegenstand der vorliegenden Erfindung ein isoliertes DNA-Fragment, das eines oder mehrere der Gene bcoC, bcoA/B und bcoD der L-Carnitin-Biosynthese umfaßt, die für eine γ-Butyrobetain-CoA-Dehydrogenase (bcoC), eine γ-Butyrobetain/Crotonobetain-CoA-Synthetase (bcoA/B) bzw. eine Crotonobetain-CoA-Hydrolase (bcoD) codieren, wobei dieses DNA-Fragment ausgewählt ist
a) aus einem 10, 6 kb-EcoRI-Fragment wie inseriert in Plasmid pVK100q, hinterlegt in Rhizobium/Agrobacterium HK 1349 unter der Hinterlegungsnummer DSM 8726, oder Subfragmenten davon; und
b) aus Fragmenten, die mit diesem 10,6 kb-EcoRI-Fragment oder Subfragmenten davon hybridisieren und für Enzyme mit den Aktivitäten einer γ-Butyrobetain-CoA-Dehydrogenase, einer γ-Butyrobetain/Crotonobetain-CoA-Synthetase, und/oder einer Crotonobetain-CoA-Hydrolase codieren,
sowie Vektoren, die diese DNA-Fragmente enthalten.

Gegenstand der Erfindung sind ferner rekombinante Mikroorganismen, die diese DNA-Fragmente bzw. Vektoren enthalten. Ein weiterer Gegenstand ist ein biotechnologisches Verfahren zur Herstellung von L-Carnitin unter Verwendung der erfindungsgemäßen Mikroorganismen.

Die Bezeichnungen bcoA/B, bcoC, bcoD und bcoT, wie sie hier in der Beschreibung und den Ansprüchen verwendet werden, umfassen definitionsgemäß sowohl die Gene von Wildtyp-Organismen, die für die Enzyme des γ-Butyrobetain/Crotonobetain-L-Carnitin-Stoffwechsels mit den oben genannten Enzymaktivitäten codieren, insbesondere die Gene des Butyrobetain-L-Carnitin (bco)-Operons, als auch deren funktionell äquivalente genetische Varianten und Mutanten, d.h. Gene, die sich von den Genen der Wildtyp-Organismen ableiten und deren Genprodukte in ihrer biologischen Funktion im wesentlichen unverändert sind. Die funktionell äquivalenten genetischen Varianten und Mutanten umfassen somit beispielsweise Basenaustausche im Rahmen der bekannten Degeneration des genetischen Codes, wie sie z. B. künstlich erzeugt werden können, um die Gensequenz an die bevorzugte Codon-Verwendung eines bestimmten Mikroorganismus, in dem eine Expression erfolgen soll, anzupassen. Die Varianten und Mutanten umfassen ferner Deletionen, Insertionen und Substitutionen von Basen oder Codons, soweit sie die Genprodukte derart veränderter Gehe in ihrer biologischen Funktion im wesentlichen unverändert lassen. Umfaßt werden hierdurch beispielsweise Gensequenzen, die zu den Wildtypsequenzen eine hohe Homologie, beispielsweise höher als 70% aufweisen und unter stringenten Hybridisierungsbedingungen, z.B. bei Temperaturen zwischen 50 und 70°C und bei 0,5 bis 1,5M Salzanteil mit dem Komplement der Wildtypsequenzen zur Hybridisierung in der Lage sind.

Unter dem Begriff Transkriptionseinheit, wie er hier verwendet wird, werden DNA-Sequenzen verstanden, in denen Gene in einer Transkriptionsrichtung angeordnet sind und unter gemeinsamer Transkriptionskontrolle in ein durchgehendes Transkript überschrieben werden, wobei die DNA-Sequenzen neben den Genen zusätzlich über zur Genexpression erforderliche genetische Kontrollelemente wie Promotoren und Ribosomenbindungsstellen verfügt.

Die Erfindung wird durch die nachfolgenden Abbildungen näher erläutert.

Fig. 1 zeigt die Enzyme des γ-Butyrobetain- bzw. des Crotonobetain-L-Carnitin-Stoffwechselweges.

Fig. 2 zeigt die Restriktionskarte eines 10,6 kb-DNA-Fragments aus Rhizobium/Agrobacterium mit dem bco-Operon.

Fig. 3 und Fig. 4 zeigen Plasmid pVK 1011 bzw. pAZ101; Pfeile geben die Lage und die Orientierung der bco-Gene und der beu-Gene (Betain-verwertende Gene; EP-A-0 543 344) an. Der Insertanteil der Plasmide ist fett dargestellt.

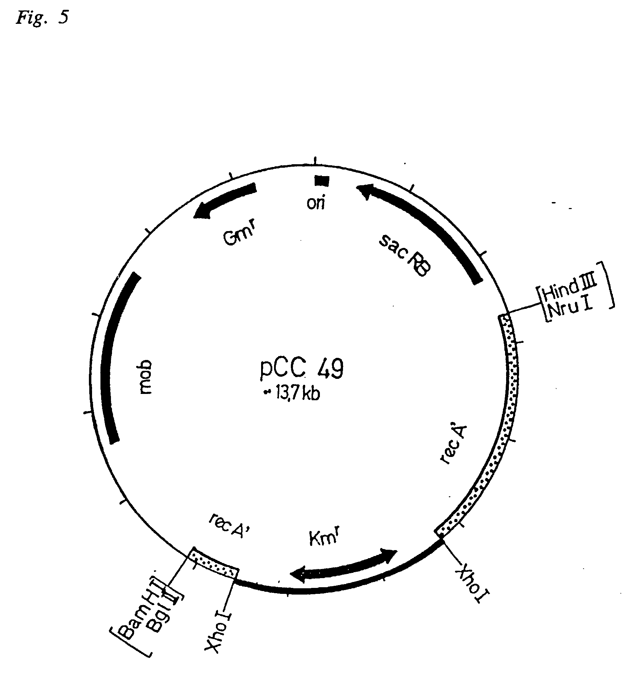

Fig. 5 zeigt Plasmid pCC49 als mögliches Ausgangsmaterial zur Herstellung eines recA⁻-Wirtsstammes.

Fig. 6 zeigt das Konstruktionsschema für die Plasmide pVK1011, pAZ101, pVK 100q und pAZ7.

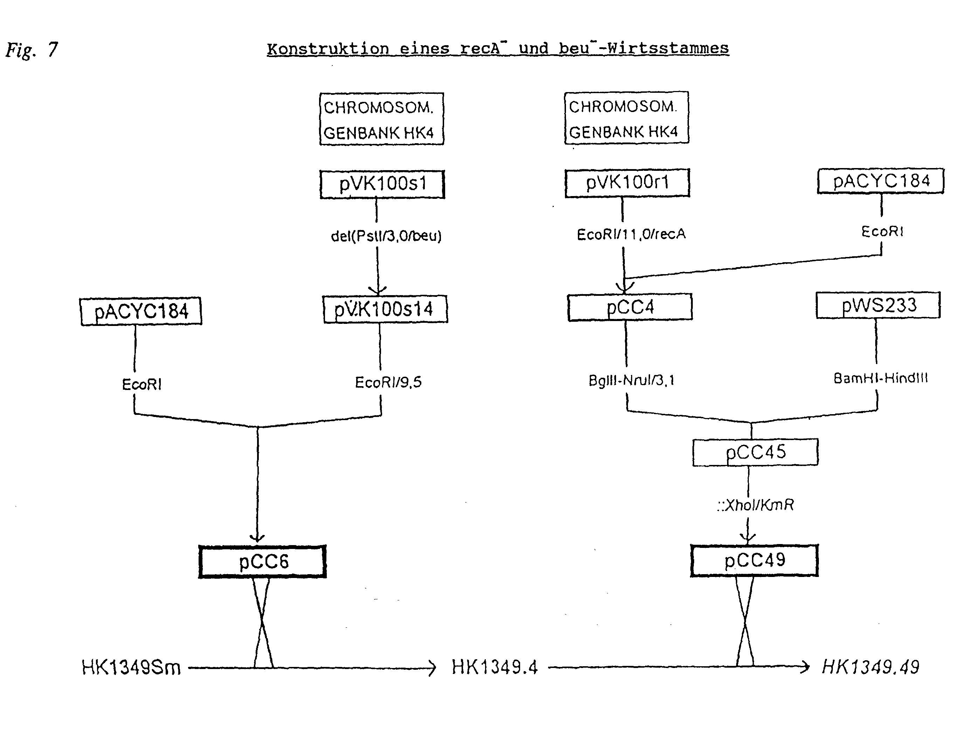

Fig. 7 zeigt ein Konstruktionsschema für einen recA⁻-Wirtsstamm.

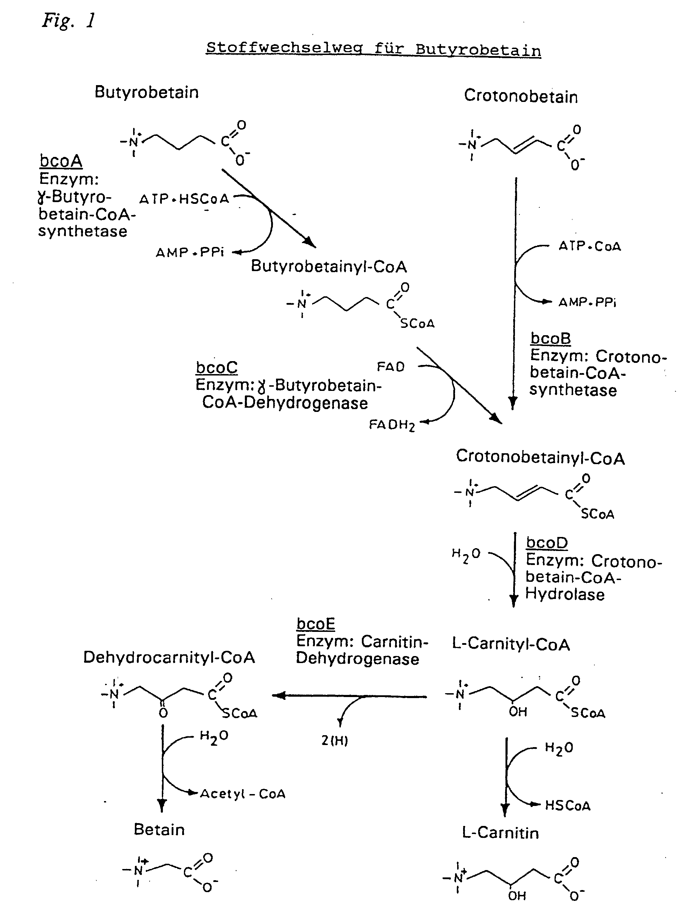

Als Ausgangsmaterial für die Isolierung der Gene bcoA/B, bcoC, bcoD und bcoT des γ-Butyrobetain-/Crotonobetain-L-Carnitin-Stoffwechselweges können alle Mikroorganismen dienen, die Butyrobetain und/oder Crotonobetain gemäß Fig. 1 verstoffwechseln. Beispiele geeigneter Stämme sind Mikroorganismen der Gattungen Escherichia, Pseudomonas, Agrobacterium, Rhizobium und Agrobacterium/Rhizobium, wobei letztere bevorzugt werden. Ein Beispiele für einen bevorzugten Mikroorganismus der Gattung Agrobacterium/Rhizobium ist ein Mikroorganismen der Spezies Agrobacterium/Rhizobium sp. HK4 (DSM 2938), wie er bereits in der EP-A-0 158 194 beschrieben wurde. Besonders bevorzugt werden Mikroorganismen verwendet, die bereits Carnitin-Dehydrogenase negativ sind, also beispielsweise kein oder nur ein defektes bcoE Gen (im folgenden auch als bcoE' bezeichnet) besitzen. Beispiele für bevorzugte Carnitin-Dehydrogenase negative Mikroorganismen sind Mikroorganismen der Spezies Agrobacterium/Rhizobium sp. HK13 (DSM 2903) und HK1331b (DSM 3225), die bereits in der EP-A-0 158 194 beschrieben sind, oder der Spezies Agrobacterium/Rhizobium sp. HK1349 (DSM 3944), der in der EP-A-0 543 344 beschrieben ist.

Die Gene bcoA/B, bcoC, bcoD und bcoT des γ-Butyrobetain/Crotonobetain-L-Carnitin-Stoffwechsels können im Chromosom eines Mikroorganismus lokalisiert werden, indem man die Mikroorganismen beispielsweise einer Transposon-Insertionsmutagenese unterwirft und dadurch die Gene des γ-Butyrobetain/Crotonobetain-L-Carnitin-Stoffwechsels mit einem geeigneten Marker, beispielsweise einer Kanamycin (Km)-Resistenz markiert. Anschließend können derart markierte Mutanten, die Intermediate des Butyrobetain-Stoffwechsels nicht mehr verwerten können, isoliert werden. Auf diese Weise können die Gene des γ-Butyrobetain/Crotonobetain-L-Carnitin-Stoffwechsels identifiziert und in ihrer Funktion zugeordnet werden. Die markierten Gene können dann kloniert und mit geeigneten Restriktionsenzymen näher charakterisiert werden. Die Isolierung der intakten Gene bzw. der erfindungsgemäßen DNA-Fragmente kann anschließend ausgehend von einer Genbank eines entsprechenden unmutierten Mikroorganismus erfolgen, aus der die bco-Gene oder Fragmente davon durch Hybridisierung mit den wie oben erhaltenen klonierten Genen des mutagenisierten Stammes in bekannter Weise isoliert und identifiziert werden können. Die erhaltenen Gene können dann in die gewünschten Vektoren kloniert und mit Hilfe von Restriktionsenzymen kartiert werden.

Für die Biosynthese von L-Carnitin sind nur die Gene bcoA/B, bcoC und bcoD verantwortlich. Dementsprechend ist für die Produktion von L-Carnitin auch nur die Anwesenheit dieser Gene erforderlich. Abhängig von den gewählten Ausgangsbedingungen, beispielsweise dem gewählten Ausgangsmaterial oder dem gewählten Produktionsstamm, können die bei der L-Carnitin-Produktion eingesetzten DNA-Fragmente und Vektoren eines oder mehrere der Gene der L-Carnitin-Biosynthese enthalten.

Neben den Genen bcoA/B, bcoC und bcoD können die erfindungsgemäßen DNA-Fragmente und Vektoren falls gewünscht auch das potentielle Transportgen bcoT umfassen.

Die Anwesenheit eines L-Carnityl-Dehydrogenase-Gens, also eines bcoE-Gens, ist unerwünscht, da in dessen Gegenwart ein Abbau von L-Carnitin erfolgt. Die Anwesenheit eines defekten bcoE-Gens (bcoE') ist jedoch unschädlich.

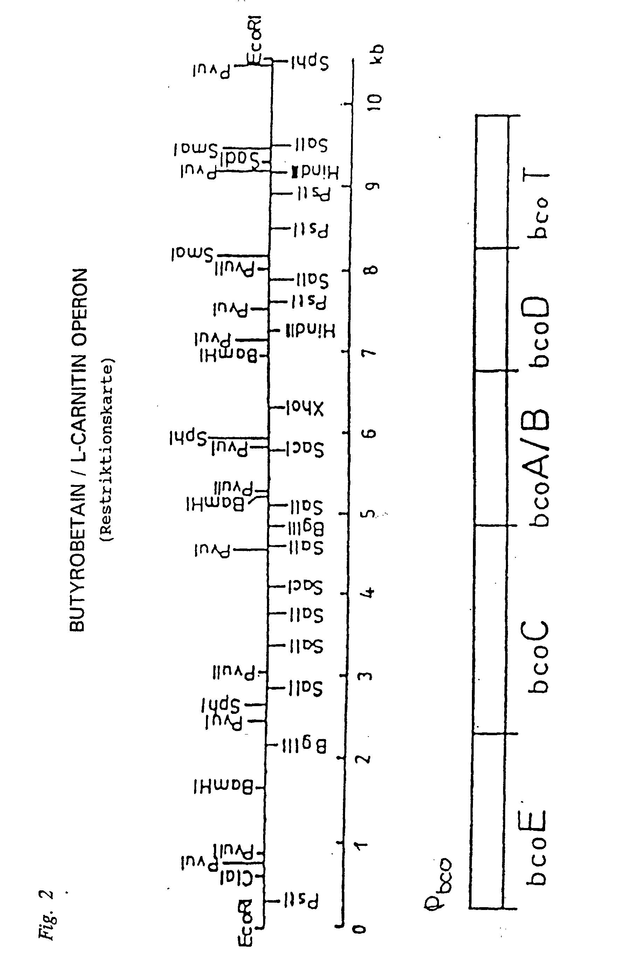

Zweckmäßig liegen die Gene für die L-Carnitin-Biosynthese, nämlich bcoC, bcoA/B und bcoD und gegebenenfalls das potentielle Transportgen bcoT, für die Anwendung bei der Produktion von L-Carnitin gemeinsam auf einem DNA-Fragment oder Vektormotung stromabwärts von den genregulatorischen Elementen, in der Reihenfolge bcoC, bcoA/B und bcoD bzw. bcoC, bcoA/B, bcoD und bcoT und in einer einzigen, wie oben definierten Transkriptionseinheit, entsprechend der Anordnung im natürlich auftretenden Butyrobetain-L-Carnitin-Operon. Die Gene bcoC, bcoA/B, bcoD und bcoT einer solchen Transkriptionseinheit können beispielsweise durch die entsprechenden Abschnitte der Restriktionskarte der Fig. 2 gekennzeichnet werden.

Die Transkription bzw. Expression der bco-Gene erfolgt zweckmäßig unter der Kontrolle eines bevorzugt starken geeigneten Promotors. Die Wahl des Promotors hängt von den gewünschten Expressionsbedingungen ab, beispielsweise davon, ob eine konstitutive oder induzierte Expression gewünscht wird, oder von dem Mikroorganismus, in dem die Expression erfolgen soll. Ein geeigneter Promotor ist beispielsweise der Promotor P_{bco} des natürlichen Butyrobetain-L-Carnitin Operons. Erfolgt die Isolierung der für die L-Carnitin-Biosynthese verantwortlichen bco-Gene beispielsweise aus einem Mikroorganismus mit einem defekten bcoE-Gen (bcoE'), so kann beispielsweise vorteilhaft das gesamte bco-Operon mit dem bcoE'-Gen und den zugehörigen genregulatorischen Elementen aus diesen Mikroorganismen isoliert und kloniert werden und anschließend zur Produktion von L-Carnitin in geeigneten Mikroorganismen eingesetzt werden. Eine derartige Transkriptionseinheit mit einem mutierten bcoE-Gen, wie sie beispielsweise aus Rhizobium/Agrobacterium HK1349 isoliert werden kann, läßt sich gegebenenfalls ebenfalls durch die in Fig. 2 wiedergegebene Restriktionskarte charakterisieren, wenn der Defekt in bcoE beispielsweise lediglich auf eine Punktmutation zurückzuführen ist und keine Restriktionsschnittstelle betrifft. Weitere zur Expression geeignete Promotoren sind beispielsweise die Promotoren P_{Nm}, P_{S1} (M. Labes et al., Gene, 89, 37-46, 1990), der trp-Promotor (Amann et al., Gene, 25, 167-178, 1983), der lac-Promotor (Amann et al. , Gene, 25, 167-178, 1983) und der tac-Promotor, ein Hybrid aus den genannten trp- und lac-Promotoren, der als konstitutiver oder induzierbarer Promotor eingesetzt werden kann (Russell und Bennett, Gene, 20, 231-243, 1982).

Zur Verwendung bei der Produktion von L-Carnitin in einem geeigneten Produktionsstamm werden die erfindungsgemäßen DNA-Fragmente, die die genannten bco-Gene, bevorzugt gemeinsam in einer einzigen Transkriptionseinheit, umfassen, zweckmäßig mit Hilfe bekannter Techniken in bekannte geeignete Vektoren, insbesondere Expressionsvektoren, beispielsweise Phagen oder Plasmide, eingebaut. Als Vektoren können autonom und selbstreplizierende Vektoren oder auch sog. Integrationsvektoren verwendet werden. Unter einem Integrationsvektor wird hierbei ein Vektor, beispielsweise ein Plasmid verstanden, welcher mindestens eine zur genomischen Sequenz des Rezipientenstammes homologe Sequenz aufweist und durch homologe Rekombination mit dieser Sequenz ein Einschleusen von Fremdgenen in das Genom des Rezipientenstamms erlaubt. Bevorzugt werden autonom und selbstreplizierende Vektoren verwendet.

Abhängig von der Art der gewählten Vektoren können die Gene für die Enzyme der L-Carnitin-Biosynthese in verschiedenen Organismen exprimiert werden. Als Vektoren eignen sich sowohl Vektoren mit spezifischem Wirtsspektrum als auch Vektoren mit breitem Wirtsspektrum ("broad host range") und die oben beschriebenen Integrationsvektoren.

Als "broad host range" Vektoren können alle Vektoren eingesetzt werden, die für Gram-negative Bakterien geeignet sind. Beispiele für solche "broad host range"-Vektoren sind pVK100 (Knauf und Nester, Plasmid, 8, 45-54, 1982), pME285 (Haas und Itoh, Gene, 36, 27-36, 1985) und pKT240 (Bagdasarian et al., Gene, 26, 273-282, 1983) oder deren Derivate. Als Derivat von pVK100 kann beispielsweise pVK1001, als Derivat von pME285 beispielsweise pAZ10 und als Derivat von pKT240 beispielsweise pLO32 (wie bereits beschrieben in der EP-A-0 543 344) verwendet werden.

Als Integrationsvektoren, im Falle von Rhizobium/Agrobacterium, können Vektoren auf der Basis von pACYC184 oder pBR322 (Comai et al., Plasmid, 10, 21-30, 1983) verwendet werden.

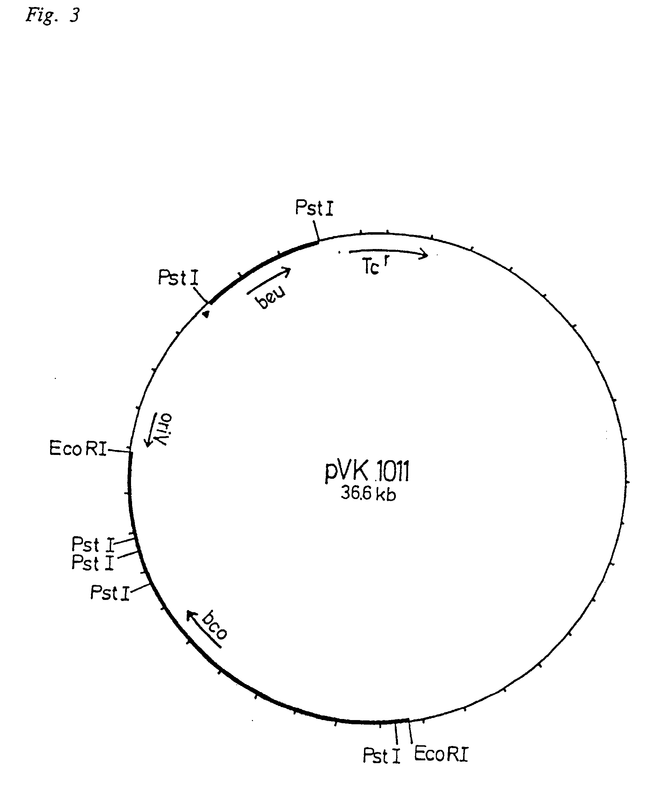

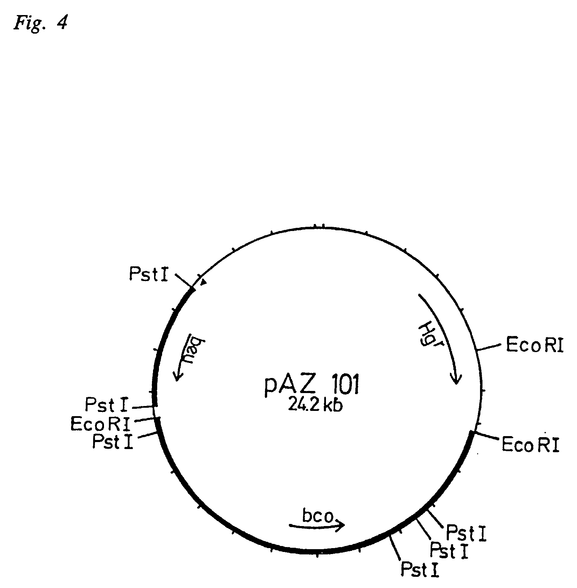

Auf diese Weise wurden beispielsweise die Plasmide pVK100q, pVK1011 (Fig. 3), pAZ7, pAZ7::beu, pAZ101 (Fig. 4) und pLO41 erhalten. Plasmid pVK100q wurde am 16.11.1993 bei der Deutschen Sammlung für Mikroorganismen und Zellkulturen GmbH, D-38124 Braunschweig, Mascheroderweg 1b, in Rhizobium/Agrobacterium HK1349 unter der Hinterlegungsnummer DSM 8726 hinterlegt.

Zur Herstellung der Produktionsstämme für die Fermentation, d.h. der Stämme für die L-Carnitin-Produktion, müssen die erfindungsgemäßen DNA-Fragmente bzw. Vektoren in die gewünschten und zur Expression geeigneten Wirtsstämme eingebracht werden. Zweckmäßig werden die Mikroorganismen hierzu in üblicher und an sich bekannter Weise mit den die erfindungsgemäßen DNA-Fragmente enthaltenden Vektoren transformiert. Die Mikroorganismen können das erfindungsgemäße DNA-Fragment dann entweder auf einem Vektormolekül oder integriert in ihrem Chromosom enthalten.

Als Produktionsstämme sind alle Mikroorganismen geeignet, die befähigt sind, aus Crotonobetain und/oder γ-Butyrobetain L-Carnitin zu produzieren und deren Fähigkeit zum Abbau (Katabolismus) von L-Carnitin ganz oder teilweise gehemmt ist. Mikroorganismen, die in ihrem L-Carnitin-Katabolismus gehemmt sind, sind beispielsweise Stämme, die Carnitin-Dehydrogenasenegativ sind, d.h. Stämme in denen das Carnitin-Dehydrogenase-Gen bcoE z.B. durch Mutation oder Deletion ausgeschaltet ist, und/oder Stämme, die L,D-Carnitin-Racemase-negativ und L-Carnitin-Dehydratase-negativ sind.

Geeignete Wirtsstämme, vorzugsweise Stämme mit hoher Substratund Edukt-Toleranz sind beispielsweise Mikroorganismen der Gattungen Escherichia, Pseudomonas, Aarobacterium, Rhizobium, Comamonas und Rhizobium/Agrobacterium, wobei letztere bevorzugt sind. Mikroorganismen der bereits oben beschriebenen Spezies Rhizobium/Agrobacterium sp. HK13, HK1331b und HK1349, sowie der Spezies HK1349.4 (wie beschrieben in der EP-A-0 543 344) sind besonders bevorzugt.

Es wurde außerdem gefunden, daß die Ausbeute an L-Carnitin noch weiter verbessert werden kann, wenn die Fähigkeit des Wirtsstamms zur Rekombination, also seine Rekombinationstendenz und Rekombinationsfrequenz, herabgesetzt ist. Dadurch wird eine Rekombination mit dem Vektor aufgrund chromosomaler Homologie eingeschränkt. Die Rekombinationsfähigkeit des Wirtsstamms kann beispielsweise in bekannter Weise durch gezielte Mutation seines recA-Gens (recA-Mutation) herabgesetzt werden. Besonders bevorzugte in ihrer Rekombinationsfähigkeit beeinträchtigte Mikroorganismen sind Mikroorganismen der spezies Rhizobium/Agrobacterium sp.. beispielsweise der erfindungsgemäß wie nachstehend beschrieben erhaltene Stämme der Spezies Rhizobium/Agrobacterium HK1349.49.

Geeignete Produktionsstämme sind somit beispielsweise Mikroorganismen der Spezies Rhizobium/Agrobacterium HK1349, HK1349.4 und HK1349.49, jeweils enthaltend Plasmid pVK100q, pVK1001, pAZ7, pAZ7::beu, pAZ101 oder pLO41.

Die Isolierung der transformierten Wirtsstämme (Produktionsstämme) kann aus einem selektiven Nährmedium erfolgen, dem ein Antibiotikum zugesetzt wird, gegen das die Stämme durch ein auf dem Vektor oder DNA-Fragment befindliches Markergen resistent ist. Werden als Produktionsstämme Mikroorganismen gemäß der EP-A-0 543 344 verwendet, d.h. Mikroorganismen, die in ihrem chromosomalen, für die Betain-Verwertung kodierenden Gen mutiert und mit einem Plasmid transformiert sind, das das für die Betain-Verwertung kodierende Gen enthält, kann auch bezüglich der Betain-Verwertung selektioniert werden. Beispiele für bezüglich der Betain-Verwertung selektionierbare Mikroorganismen sind die bereits erwähnten HK1349.4 und HK1349.49, die beispielsweise Plasmid pLO41, pAZ101, pAZ7::beu oder pVK1011 enthalten.

Die biotechnologische Herstellung von L-Carnitin erfolgt unter Verwendung der Mikroorganismen, die die erfindungsgemäßen DNA-Fragmente bzw. Vektoren enthalten. Das Verfahren zur Herstellung von L-Carnitin erfolgt nach an sich bekannten Methoden, z.B. wie in der EP-A-0 158 194 beschrieben, ausgehend von z.B. γ-Butyrobetain in Gegenwart einer geeigneten Kohlenstoff- und Stickstoff-Quelle. Als Kohlenstoff- und Stickstoff-Quelle können beispielsweise Glutamat, Acetat und Betain, oder Glycerin und Betain eingesetzt werden. Wird die biotechnologische Herstellung mittels bezüglich der Betain-Verwertung selektionierbarer Mikroorganismen durchgeführt, wird als einzige Stickstoffquelle Betain verwendet.

Die Fermentation und die anschließende Isolation von L-Carnitin kann analog zu dem in der EP-A-0 158 194 beschriebenen Verfahren erfolgen.

Durch Variation der Nährstoffe im Medium und durch Anpassen der Fermentationsbedingungen an den jeweiligen Mikroorganismus in üblicher Weise kann die Ausbeute an L-Carnitin weiter verbessert werden.

### BEISPIEL 1

### Erzeugung von Transposon-Insertionsmutanten (Tn5) und ihre phänotypische Identifizierung

Der Wildtyp-Stamm Rhizobium-/Agrobacterium HK4 (DSM 2938, EP-B 0 158 194) wurde durch Selektionsdruck zur Entwicklung einer Spontanresistenz gegenüber Streotomycin (Sm, 1000 µg/ml) gebracht. Diese Resistenz war nachweisbar über 50 Generationen ohne Selektion stabil und wurde als Selektionsmarker verwendet.

0,2 ml einer Tn5-Donorkultur, von E. coli S17-1/pSUP2021 (R.

Simon et al., Biotechnology, 1983, 1, 784-790), wurden mit 2 ml der Rezipientenkultur HK4 vermischt und zentrifugiert. Die Zellen wurden in 0,9% Saline (NaCl-Lösung) gewaschen und in 100 µl 0,9% Saline resuspendiert. Die Konjugation des Rezipientenstammes mit dem Donorstamm erfolgte über Nacht bei 30°C auf trockenem Nutrientagar. Die geernteten Zellen wurden in Verdünnungen auf Selektionsmedium für Rezipient (Sm^{R}) und Transposon (Neomycin-Resistenz (Nm^{R})) plattiert.

Tn5-Mutanten wurden auf Nutrientagar mit Sm (1000 µg/ml) und Nm (100 µg/ml) erhalten. Die phänotypische Identifizierung der Mutanten gelang über den Nachweis der Nichtverwertung der Butyrobetain-Stoffwechselintermediaten gemäß Fig. 1 als Kohlenstoff (C-)-Quelle in Minimalmedium.

### BEISPIEL 2

### Klonierung der Tn5-markierten DNA-Fragmente aus dem HK4-Genom

Isolierte genomische DNA von Tn5-mutiertem HK4 (5 µg) wurde vollständig mit EcoRI (4 U/µg) verdaut. pBR325 (2,5 µg) (Gene, 1977, 2, 95-113) wurde nach vollständiger Verdauung durch EcoRI mit alkalischer Phosphatase behandelt. Rekombinante Hybridplasmide wurden nach Vermischen von genomischer DNA und pBR325 mit T4-DNA-Ligase (0,2 U (Units)/µgDNA) in 400 µl Ligationspuffer (20 mM Tris-HCl, pH 7,2, 10 mM DTT (Dithiothreitol), 10 mM MgCl₂, 0,6 mM ATP) und Inkubation über Nacht bei 12°C erhalten.

Aliquots des Ligationsgemisches wurden zur Transformation (Cohen et al., 1972, PNAS 69, 2110-2114) von E.coli ED 8654 (Barek et al., Mol. Gen. Genet., 146, 199 - 207, 1976) eingesetzt. Transformanten wurden in Nutrientmedium auf ihre Resistenz gegen Ampicillin (Ap, 100 µg/ml) und Kanamycin (Km, 25 µg/ml) selektioniert. Alle selektionierten Hybridplasmide trugen ein HK4-Insert, welches mit Tn5 markiert war. Die Inserts wurden für verschiedene Restriktionsenzyme, entsprechend der Restriktionskarte in Fig. 2, kartiert. Vergleiche der Restriktionskarten belegten die Transposoninsertion im gleichen genomischen Fragment bei einer Reihe von Tn5-Mutanten mit unterschliedlichem Phänotyp.

Bestätigung dieser Beobachtung konnte durch Southern Blot-Hybridisierung ("Gentechnische Methoden", herausgegeben von S. Bertram und H.G. Gassen, G. Fischer Verlag 1991, 219f.) identisch geschnittener Plasmid-DNA und anschließender elektrophoretischer Auftrennung erhalten werden. Als Sonden dienten Subfragmente dieser klonierten DNA aus den Transposon-Mutanten.

### BEISPIEL 3

### Erstellen einer genomischen HK4-Genbank in Lambda-Phagen

Damit DNA in Lambda-Phagen verpackt werden kann, braucht sie eine Größe von 40 - 52 kb und eine "cos-site". Zum Erstellen einer genomischen HK4-Genbank in Lambda-Phagen wurde der Cosmidvektor pVK100 (Knauf und Nester, 1982, Plasmid 8, 45-54) verwendet, der 23 kb groß ist und somit die Klonierung von DNA-Fragmenten zwischen 17-29 kb gestattet.

pVK100 wurde mit EcoRI verdaut, dephosphoryliert und mit HK4-DNA, die partiell mit EcoRI verdaut war, ligiert. Die ideale EcoRI-Konzentration für den Partialverdau von HK4-DNA wurde über Testverdaus als 0,58 U/µg DNA bzw. 0,02 U/µl Reaktionsgemisch ermittelt, wobei 8,5 µg HK4-DNA im Reaktionsgemisch eingesetzt wurden. Die DNA-Fragmente im Größenbereich von größer als 17 kb wurden aus Agarose-Elektrophoresegelen isoliert. Die Ligation erfolgte in 10 µl Volumina mit einem Gehalt an 100 ng Cosmid-Vektor und 400 ng Passenger-DNA. Anschließend erfolgte "In vitro-Packaging" entsprechend dem Protokoll des Herstellers im Mix der Fa. Promega Biotec. innerhalb 2 Stunden bei 25°C. Nach Transfektion von E. coli S17-1 wurde auf die Km-Resistenz des Cosmid-Vektors selektioniert. Ca. 5500 Kolonien (individuelle Klone) wurden mit einem Ansatz erhalten. Die Genbank-Kolonien wurden in 5 "batches" von je ca. 1000 Klonen in Gefriermedium (Nutrient Yeast Broth, NYB, Oxoid und 50% Glycerin) bei -70°C aufbewahrt. Die Amplifizierung der Genbank erfolgte mit je 50 µl dieser batches in 10 ml NYB-Übernachtkultur und Portionierung.

### BEISPIEL 4

### Screening der HK4-Cosmid-Genbank, Identifizierung der bco-Gene-tragenden Cosmidklone über Kolonienhybridisierung, Dot-Blotting-Hybridisierung oder direkte Komplementierung an HK-Mutanten

Als Hybridisierungssonden konnten direkt die klonierten, Tn5-markierten DNA-Fragmente eingesetzt werden.

Klone mit entsprechenden DNA-Sequenzen zeigten Hybridisierungssignale und führten zur Komplementierung des jeweils defekten Gens in der HK4-Mutante. Kreuzhybridisierungen der DNA aus verschiedenen Mutanten belegten die "Clusterung" mehrerer Gene des Butyrobetain-Stoffwechsels auf einem DNA-Fragment von 10,6 kb (Fig. 2).

Die Kolonienhybridisierung wurde in üblicher Weise (S. Bertram und H.G. Gassen, 1991, ibid, 221f.) durchgeführt. Dot-Blotting wurde ebenfalls in bekannter Weise (S. Bertram und H.G. Gassen, 1991, ibid, 217f.) durchgeführt.

### BEISPIEL 5

### Komplementierung von HK-Mutanten

Nach genauer Lokalisation der einzelnen Gene des Butyrobetain-Stoffwechsels unter Berücksichtigung der molekualren Größen aufgrund der identifizierten Peptidketten konnte eine Komplementierung der einzelnen Mutanten durch die jeweiligen Gen-Abschnitte erzielt werden.

Das jeweilige Expressionsplasmid pVK100::HK-DNA wurde via Konjugation aus E. coli S17-1 in Rhizobium/Agrobacterium sp. HK4-Stämme gemäß Beispiel 1, die Mutationen für unterschiedliche Stoffwechselschritte aufwiesen (siehe Fig. 1), eingebracht. Selektion erfolgte gegen die Prolin (pro)-Auxotrophie des Donors und auf die Antibiotikaresistenz des Vektors (Km^{R}Tc (Tetracyclin)^{R}).

### BEISPIEL 6

### 6.1 Klonierung des bco-Fragments aus Stamm HK1349 (DSM 3944) und Abkömmlinge

Zur Erzielung von Gendosis-Effekten und Produktivitätssteigerungen am Produktionsstamm wurde das bco-Operon aus HK 1349 (DSM 3944, EP-A-0 543 344) kloniert. In diesem Stamm ist das gesamte bco-Operon komplett enthalten, jedoch ist das erste Gen, bcoE, für die Carnitin-CoA-Dehydrogenase mutiert. Ein aus diesem Stamm erhaltenes DNA-Fragment mit dem bco-Operon ist somit ideal für Produktivitätssteigerungen aufgrund hoher Kopienzahlen der Expressionsvektoren.

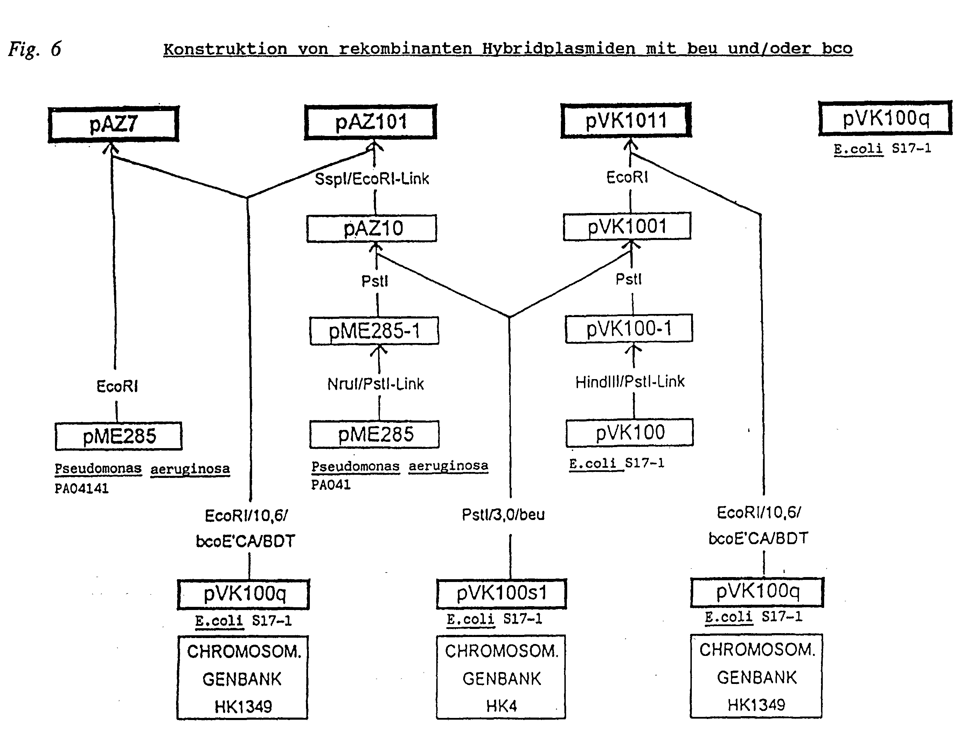

Die Klonierungen erfolgten in bekannter Weise in E. coli S17-1, entsprechend zu Beispiel 3 der EP-A-0 543 344. Hierzu wurde das 10,6 kb große bco-Operon aus einer HK1349-Genbank isoliert und in pVK100 ligiert. Daraus resultierte Plasmid pVK100q (siehe Konstruktionsschema gemäß Fig. 6). Selekioniert wurde in E. coli S17-1 auf NYB Km (25 µg/ml). Die Identifikation des richtigen Inserts gelang gemäß der in Beispiel 5 beschriebenen Methode an HK-Mutanten. EcoRI geschnittene DNA von HK1349 wurde elektrophoretisch aufgetrennt und die Fragmente im Größenbereich von 10,6 kb wurden aus dem Gel isoliert. Die isoleirten Fragmente wurden in EcoRI geschnittenen pVK100 ligiert. Mit diesem Hybridplasmid-Gemisch wurde E. coli S17-1 (Prolin-Auxotroph) transformiert und auf Nutrient Agar Km (25 µg/ml) selektioniert. Die Identifikation des richtigen Hybridplasmid-Klons aus Vektor und dem das bco-Operon tragenden 10,6 kb DNA-Fragment erfolgte über "patch-mating" auf einem Rasen einer Butyrobetain-CoA-Synthetase-negativen Mutante (HK4V4) auf Minimalmedium mit 0,2% (w/v) Butyrobetain als C- und N-Quelle. Der richtige Klon vermochte nach derartigem konjugativen Transfer in die Mutante die Zellen in der Verwertung dieser C-Quelle zu komplementieren. Der so identifizierte Klon pVK100q diente direkt als Produktionsplasmid oder als Reservoir für das DNA-Fragment mit dem bco-Operon für weitere Subklonierungen.

### 6.2 Konstruktion von pAZ101, pAZ7, pVK1011 und pVK100q, pLO41 und pAZ7::beu

Die Konstruktion von pAZ101, pAZ7, pVK1011 und pVK100q erfolgte gemäß dem Konstruktionsschema von Fig. 6. Die Konstruktion von pLO41 erfolgte ausgehend von pLO32 (EP-A-0 543 344) durch Insertion der bco-Gene (10,6 kb großes EcoRI/EcoRI-Fragment) und die Konstruktion von Plasmid pAZ7::beu ausgehend von pAZ7 (Fig. 6) durch Insertion der beu-Gene (3 kb großes PstI/PstI-Fragment, EP-A-0 543 344). Die entsprechenden Restriktionsenzyme wurden mit 3 - 5 U/µg DNA nach den Angaben der Hersteller eingesetzt. Plasmid pVK100q wurde durch Insertion der bco-Gene in pVK100 (Knauf und Nester; ibid) erhalten. Das Ausgangsplasmid pVK100s1 (Konstruktionsschema Fig. 6) wurde durch EcoRI-Deletionsklonierungen von Plasmid pVK100s (EP-A-0 543 344) erhalten.

### BEISPIEL 7

### Einführung einer recA-Mutation in HK1349.4

### 7.1 Identifikation des für recA codierenden Genbank-Klons

Mit Hilfe der Methode der Kolonienhybridisierung (S. Bertram und H.G. Gassen, 1991, ibid, 221f.) wurde die genomische HK4-Cosmid-Genbank auf recA-codierende Klone untersucht. Als Sonde zur Hybridisierung diente das klonierte recA-Gen aus Rhizobium leguminosarum (W. Selbitschka et al., Mol. Gen. Genet., 299, 1991, 86-95). Der markierte Cosmidklon wurde isoliert, und die nach EcoRI-Verdau erhaltenen EcoRI-DNA-Insertfragmente wurden mittels Southern Blot-Hybridisierung gegen die recA-Sonde (S. Bertram und H.G. Gassen, 1991, ibid, 219f.) auf recA-homologe Sequenzen untersucht. Das so markierte EcoRI-Fragment wurde in den gleichermaßen geschnittenen Vektor pVK100 ligiert. Mit dem resultierenden Hybridplasmid pVK100r1 wurden zur DNA-Vermehrung E. coli S17-1-Zellen transformiert.

### 7.2. Einführung eines Kanamycin-Resistenzgens in HK1349.4 zur Inaktivierung des chromosomalen recA-Gens

Das 11,0 kb große EcoRI-Fragment wurde aus pVK100rl in pACYC184 (A.C.Y. Chang und S.N. Cohen, J. Bacteriol., 134, 1978, 1141-1156), der mit EcoRI geschnitten war, umkloniert.

Entsprechend der ermittelten Restriktionskarte wurde ein BglII-NruI geschnittenes 3,1 kb großes Subfragment, welches bei Southern Blot-Hybridisierung gegen die recA-Sonde markiert wurde, in den Vektor pWS233, BamHI-HindIII geschnitten, kloniert. pWS233 ist ein "gene replacement" Vektor und bei W. Selbitschka et al., Appl. Microbiol. Biotechnol. 38, 1993, 615 - 618, beschrieben.

In die XhoI-Schnittstelle des resultierenden Plasmids pCC45 wurde ein XhoI-DNA-Fragment aus Tn5 (pSUP2021, R. Simon et al., Biotechnology, 1, 1983, ibid) einkloniert, welches für Km-Resistenz codiert. Daraus resultierte pCC49. Analog dem Vorgehen von W. Selbitschka et al., 1993, ibid, wurde ein "gene replacement" auf folgende Weise durchgeführt:

3 ml exponentieller HK1349.4-Kultur wurden mit 1 ml exponentieller Donorkultur (S17-1/pCC49) zusammengegeben, zentrifugiert und mit 0,9% NaCl-Lösung gewaschen. Die Zellen wurden in 50 µl NYB auf Nutrientagar über Nacht bei 30°C inkubiert. Anschließend wurden die in 0,9% NaCl-Lösung resuspendierten Zellen in geeigneter Verdünnung auf Selektivmedien aufgebracht. Transkonjuganten des Sm-resistenten Rezipienten HK1349.4 wurden auf Nutrientagar mit Sm (1000 µg/ml) und Nm (150 µg/ml) erhalten und zeigten entsprechend den Genen sacRB auf dem "replacement" Vektor Sensitivität gegenüber 5% Saccharose im Komplexmedium.

Doppelte Rekombinationen wurden nach Kultivierung der selektionierten Transkonjuganten ohne Selektiorisdruck auf Nutrientagar mit geringer Frequenz (10⁻⁸) erhalten: Nach ca. 1 Woche konnten Zellen isoliert werden, welche 5% Saccharose im Medium tolerierten, Nm-resistent geblieben waren, aber wieder Gentamycin (Gm)-sensitiv (Vektormarker) geworden waren.

Dieser Phänotyp bestätigt den allelen Markeraustausch und deutet auf eine recA-Mutation in HK1349.4. Der typische Phänotyp (z.B. UV-Sensitivität etc.) von recA-Mutanten ist beobachtbar.

In dem auf diese Weise erhaltenen Stamm HK1349.49 ist die Tendenz der chromosomalen Integration von Plasmiden mit homologen Sequenzen (homologe Rekombination) deutlich herabgesetzt. Der Stamm ist daher ein gutgeeigneter Wirt für die in Beispiel 6.2 dargestellten Hybridplasmide.

### BEISPIEL 8

### Biotransformation

Die Biotransformation wrude in Schüttelkolben (100 ml) mit N-freiem Minimalmedium (MM; Kulla et al., Arch. Microbiol., 1983, 135, S. 1-7) durchgeführt, enthaltend 0,2% (w/v) γ-Butyrobetain (Edukt) und als C-Quelle 0,4% (w/v) Glycerin. Als N-und zusätzliche C-Quelle wurde 0,2% (w/v) L-Glutamat (bei in der Betainverwertung negativen Stämmen) oder 0,2% (w/v) Betain zugegeben.

Als Produktionsstämme wurden die erfindungsgemäßen Stämme HK1349.4/pLO41, HK1349.4/pVK1011, HK1349.4/pAZ7::beu, HK1349.4/pAZ101, HK1349/pVK100q und HK1349/pAZ7 eingesetzt.

Die Ergebnisse sind in Tabelle 1 zusammengefaßt im Vergleich zu den aus der EP-A-0 543 344 bekannten HK13-Abkömmlingen HK1349 (DSM 3944) und HK1349.4.

Die Bildung von L-Carnitin aus γ-Butyrobetain wurde nach der bei Bergmeyer beschriebenen DTNB (5-5'-Dithiobis-(2-nitrobenzoat)-Methode bestimmt (H.K. Bergmeyer, 1974, Methoden der enzymatischen Analyse, Verlag Chemie, Weinheim, 1810f.).

| Stämme | Medium MM & Glycerin | spez.Aktivität mmol/1/h/OD |
|---|---|---|
| HK1349 (nicht erfindungsgemäss) | & Betain | 0,24 |
| HK1349 / pVK 100q | & Betain | 0,36 |
| HK1349 / pAZ7 | & Betain | 0,49 |
| HK1349 (nicht erfindungsgemäss) | & L-Glutamat | 0,14 |
| HK1349.4 (nicht erfindungsgemäss) | & L-Glutamat | 0,11 |
| HK1349.4 / pLO41 | & L-Glutamat | 0,29 |
| HK1349.4 (nicht erfindungsgemäss) | & Ammonium | 0,12 |
| HK1349.4 / pVK1011 | & Betain | 0,54 |
| HK 1349.4 / pAZ7::beu | & Betain | 0,47 |
| HK1349.4 / pAZ101 | & Betain | 1,08 |

## Patentansprüche

1. Isoliertes DNA-Fragment umfassend eines oder mehrere der Gene bcoC, bcoA/B und bcoD der L-Carnitin-Biosynthese, die für eine γ-Butyrobetain-CoA-Dehydrogenase (bcoC), eine γ-Butyrobetain/Crotonobetain-CoA-Synthetase (bcoA/B) bzw. eine Crotonobetain-CoA-Hydrolase (bcoD) codieren, wobei dieses DNA-Fragment ausgewählt ist
a) aus einem 10,6 kb-EcoRI-Fragment wie inseriert in Plasmid pVK100q, hinterlegt in Rhizobium/Agrobacterium HK 1349 unter der Hinterlegungsnummer DSM 8726, oder Subfragmenten davon; und
b) aus Fragmenten, die mit diesem 10,6 kb-EcoRI-Fragment oder Subfragmenten davon hybridisieren und für Enzyme mit den Aktivitäten einer γ-Butyrobetain-CoA-Dehydrogenase, einer γ-Butyrobetain/Crotonobetain-CoA-Synthetase, und/oder einer Crotonobetain-CoA-Hydrolase codieren.

2. DNA-Fragment nach Anspruch 1, zusätzlich umfassend das dem γ-Butyrobetain/Crotonobetain-Stoffwechsel zugeordnete, für ein potentielles Transportprotein codierende Gen bcoT.

3. DNA-Fragment nach irgendeinem der Ansprüche 1 und 2, worin sich die Gene bcoC, bcoA/B, bcoD und gegebenenfalls bcoT aus Mikroorganismen der Gattungen Escherichia, Pseudomonas, Agrobacterium, Rhizobium und Rhizobium/Agrobacterium ableiten.

4. DNA-Fragment nach irgendeinem der Ansprüche 1 bis 3, worin die Gene operativ mit zur Expression erforderlichen genetischen Kontrollelementen verknüpft sind.

5. DNA-Fragment nach irgendeinem der Ansprüche 1 bis 4, worin die Gene der L-Carnitin-Biosynthese in der Reihenfolge bcoC, bcoA/B und bcoD oder, gegebenenfalls, bcoC, bcoA/B, bcoD und bcoT angeordnet sind und als eine einzige Transkriptionseinheit vorliegen.

6. DNA-Fragment nach irgendeinem der Ansprüche 4 und 5, worin das genregulatorische Element den Promotor des natürlichen bco-Operons, P_{bco}, umfaßt.

7. DNA-Fragment nach irgendeinem der Ansprüche 1 bis 6, worin die Gene bcoC, bcoA/B, bcoD und bcoT durch die nachstehende Restriktionskarte gekennzeichnet sind:

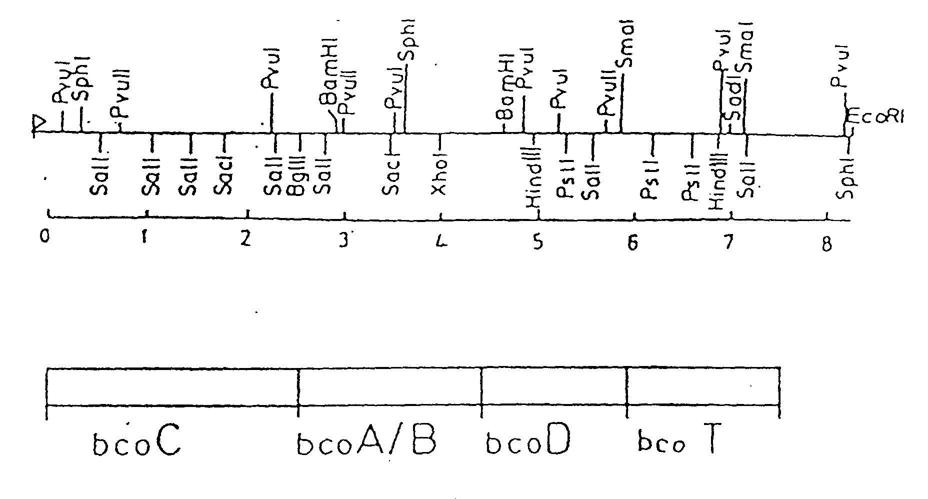

8. Vektor, enthaltend ein DNA-Fragment nach irgendeinem der Ansprüche 1 bis 7.

9. Vektor nach Anspruch 8, nämlich Plasmid pVK100q, wie hinterlegt in Rhizobium/Agrobacterium HK 1349 unter der Hinterlegungsnummer DSM 8726, Plasmid pVK1011, wie **gekennzeichnet durch** die Restriktionskarte gemäß Fig. 3, und Plasmid pAZ101, wie **gekennzeichnet durch** die Restriktionskarte gemäß Fig. 4.

10. Rekombinanter Mikroorganismus, enthaltend ein DNA-Fragment oder einen Vektor nach irgendeinem der Ansprüche 1 bis 9.

11. Mikroorganismus nach Anspruch 10, **dadurch gekennzeichnet, daß** seine Fähigkeit zum Katabolismus von L-Carnitin ganz oder teilweise gehemmt ist.

12. Mikroorganismus nach irgendeinem der Ansprüche 10 und 11, **dadurch gekennzeichnet, daß** seine Fähigkeit zur Rekombination beeinträchtigt ist.

13. Mikroorganismus nach irgendeinem der Ansprüche 10 bis 12, ausgewählt aus Mikroorganismen der Gattungen Escherichia, Pseudomonas, Agrobacterium, Rhizobium, Comamonas und Rhizobium/Agrobacterium.

14. Mikroorganismus Rhizobium/Agrobacterium HK 1349, enthaltend Plasmid pVK100q, wie hinterlegt unter der Hinterlegungsnummer DSM 8726, Plasmid pVK1011, wie **gekennzeichnet durch** die Restriktionskarte gemäß Fig. 3, oder Plasmid pAZ101, wie **gekennzeichnet durch** die Restriktionskarte gemäß Fig. 4, sowie von solchen Mikroorganismen abgeleitete genetische Varianten und Mutanten mit der Fähigkeit zur L-Carnitin-Biosynthese.

15. Mikroorganismus Rhizobium/Agrobacterium HK 1349.4, enthaltend Plasmid pVK100q, wie hinterlegt in Rhizobium/Agrobacterium HK 1349 unter der Hinterlegungsnummer DSM 8726, Plasmid pVK1011, wie **gekennzeichnet durch** die Restriktionskarte gemäß Fig. 3, oder Plasmid pAZ101, wie **gekennzeichnet durch** die Restriktionskarte gemäß Fig. 4, sowie von solchen Mikroorganismen abgeleitete genetische Varianten und Mutanten mit der Fähigkeit zur L-Carnitin-Biosynthese.

16. Biotechnologisches Verfahren zur Herstellung von L-Carnitin, **dadurch gekennzeichnet, daß** man Crotonbetain und/oder γ-Butyrobetain in Gegenwart einer geeigneten Kohlenstoff- und Stickstoffquelle mittels eines Mikroorganismus nach irgendeinem der Ansprüche 10 bis 15 fermentiert und das L-Carnitin isoliert.

## Claims

1. Isolated DNA fragment comprising one or more of the genes bcoC, bcoA/B and bcoD of the L-carnitine biosynthesis coding for a γ-butyrobetaine-CoA-dehydrogenase (bcoC), a γ-butyrobetaine/crotonobetaine-CoA-synthetase (bcoA/B) and a crotonobetaine-CoA-hydrolase (bcoD), respectively, said DNA fragment being selected from
a) a 10.6 kb EcoRI fragment as inserted into plasmid pVK100q, deposited in Rhizobium/Agrobacterium HK 1349 under the deposit number DSM 8726, or subfragments thereof; and
b) fragments hybridizing with this 10.6 kb EcoRI fragment or subfragments thereof and coding for enzymes with the activity of a γ-butyrobetaine-CoA-dehydrogenase, a γ-butyrobetaine/crotonobetaine-CoA-synthetase and/or a crotonobetaine-CoA-hydrolase.

2. DNA fragment according to Claim 1, further comprising the gene bcoT coding for a potential transport protein assigned to γ-butyro-betaine/crotonobetaine metabolism.

3. DNA fragment according to any one of Claims 1 and 2, in which the genes bcoC, bcoA/B, bcoD and optionally bcoT are derived from microorganisms of the genera Escherichia, Pseudomonas, Agrobacterium, Rhizobium and Rhizobium/Agrobacterium.

4. DNA fragment according to any one of Claims 1 to 3, in which the genes are operatively linked with the genetic control elements necessary for expression.

5. DNA fragment according to any one of Claims 1 to 4, in which the genes of L-carnitine biosynthesis are arranged in the sequence bcoC, bcoA/B and bcoD or, if appropriate, bcoC, bcoA/B, bcoD and bcoT and are present as a single transcription unit.

6. DNA fragment according to any one of Claims 4 and 5, in which the gene-regulatory element comprises the promoter of the natural bco operon P_{bco.}

7. DNA fragment according to any one of Claims 1 to 6, in which the genes bcoC, bcoA/B, bcoD and bcoT are characterized as shown in the restriction map below:

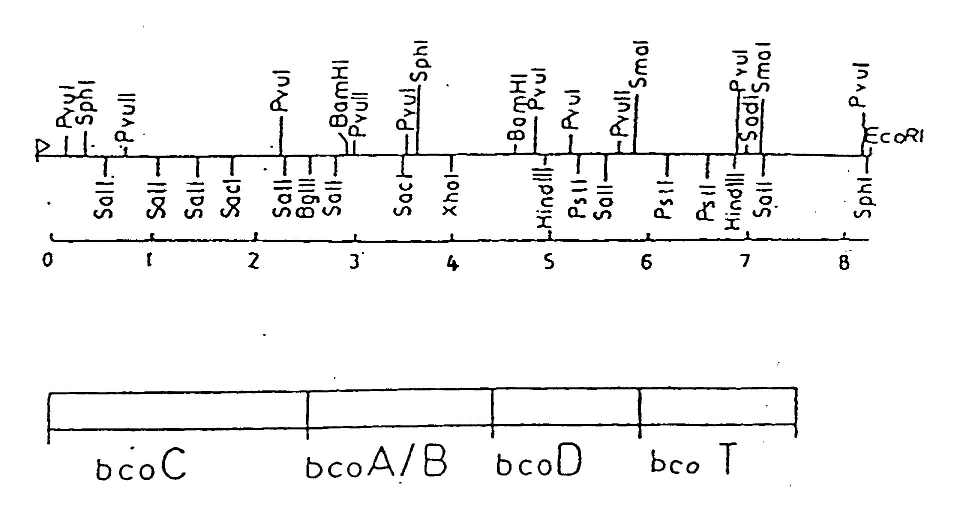

8. Vector comprising a DNA fragment according to any one of Claims 1 to 7.

9. Vector according to Claim 8, namely plasmid pVK100q, as deposited in Rhizobium/Agrobacterium HK 1349 under the deposit number DM 8726, plasmid pVK1011, as **characterized by** the restriction map according to Fig. 3, and plasmid pAZ101, as **characterized by** the restriction map according to Fig. 4.

10. Recombinant microorganism, containing a DNA fragment or a vector according to any one of Claims 1 to 9.

11. Microorganism according to Claim 10, **characterized in that** its capability to catabolize L-carnitine is completely or partially inhibited.

12. Microorganism according to any one of Claims 10 and 11, **characterized in that** its capability for recombination is impaired.

13. Microorganism according to any one of Claims 10 to 12, selected from microorganisms of the genera Escherichia, Pseudomonas, Agrobacterium, Rhizobium, Comamonas and Rhizobium/Agrobacterium.

14. Microorganism Rhizobium/Agrobacterium HK 1349, containing plasmid pVK100q, as deposited under the deposit number DMS 8726, plasmid pVK1011, as **characterized by** the restriction map according to Fig. 3, or plasmid pAZ101, as **characterized by** the restriction map according to Fig. 4, as well as genetic variants and mutants derived from such microorganisms having the capability for L-carnitine biosynthesis.

15. Microorganism Rhizobium/Agrobacterium HK 1349.4, containing plasmid pVK100q, as deposited under the deposit number DMS 8726, plasmid pVK1011, as **characterized by** the restriction map according to Fig. 3, or plasmid pAZ101, as **characterized by** the restriction map according to Fig. 4, as well as genetic variants and mutants derived from such microorganisms having the capability for L-carnitine biosynthesis.

16. Biotechnological process for the production of L-carnitine, **characterized in that** crotonobetaine and/or γ-butyrobetaine is fermented in the presence of a suitable carbon and nitrogen source by means of a microorganism according to any one of Claims 10 to 15 and L-carnitine is isolated.

## Revendications

1. Fragment d'ADN isolé comprenant un ou plusieurs des gènes bcoC, bcoA/B et bcoD de la biosynthèse de la L-carnitine qui codent pour une γ-butyrobétaïne-CoA-déshydrogénase (bcoC), une γ-butyrobétaïne/crotonobétaïne -CoA-synthétase (bcoA/B) ou une crotonobétaïne-CoA-hydrolase (bcoD), ce fragment d'ADN étant sélectionné parmi
a) un fragment EcoRI de 10,6 kb tel qu'il est inséré dans le plasmide pVK100q, déposé dans Rhizobium/Agrobacterium HK 1349 sous le numéro d'enregistrement DSM 8726, ou ses sous-fragments ;
b) des fragments qui s'hybrident avec ce fragment EcoRI de 10,6 kb ou ses sous-fragments et qui codent pour les enzymes présentant les activités d'une γ-butyrobétaïne-CoA-déshydrogénase, d'une γ-butyrobétaïne/crotonobétaïne-CoA-synthétase et/ou d'une crotonobétaïne-CoA-hydrolase.

2. Fragment d'ADN selon la revendication 1, comprenant en outre le gène bcoT attribué au métabolisme de la γ-butyrobétaïne/crotonobétaïne et codant pour une protéine de transport potentielle.

3. Fragment d'ADN selon l'une quelconque des revendications 1 et 2, dans lequel les gènes bcoC, bcoA/B, bcoD et le cas échéant, bcoT découlent de microorganismes des genres Escherichia, Pseudomonas, Agrobacterium, Rhizobium et Rhizobium/Agrobacterium.

4. Fragment d'ADN selon l'une quelconque des revendications 1 à 3, dans lequel les gènes sont liés de manière fonctionnelle ou opérative aux éléments génétiques de contrôle nécessaire à l'expression.

5. Fragment d'ADN selon l'une quelconque des revendications 1 à 4, dans lequel les gènes de la biosynthèse de la L-carnitine sont placés dans l'ordre bcoC, bcoA/B et bcoD ou le cas échéant, bcoC, bcoA/B, bcoD et bcoT et constituent une seule unité de transcription.

6. Fragment d'ADN selon l'une quelconque des revendications 4 et 5, dans lequel l'élément de régulation génétique comprend le promoteur de l'opéron naturel de bco, P_{bco}.

7. Fragment d'ADN selon l'une quelconque des revendications 1 à 6, dans lequel les gènes bcoC, bcoA/B, bcoD et bcoT sont **caractérisés par** la carte de restriction suivante :

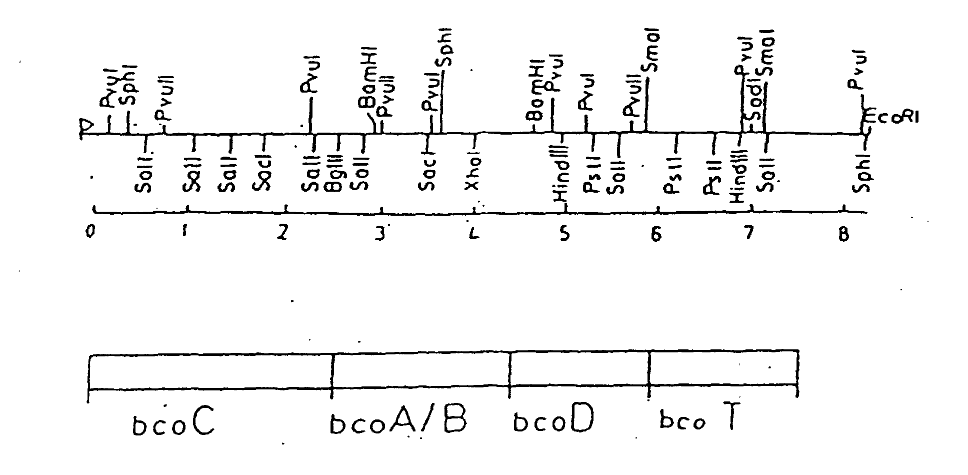

8. Vecteur comprenant un fragment d'ADN selon l'une quelconque des revendications 1 à 7.

9. Vecteur selon la revendication 8, à savoir le plasmide pVK100q tel qu'il est enregistré dans Rhizobium/Agrobacterium HK 1349 sous le numéro d'enregistrement DSM 8726, le plasmide pVK1011, **caractérisé par** la carte de restriction selon la figure 3, et le plasmide pAZ101, **caractérisé par** la carte de restriction selon la figure 4.

10. Microorganisme recombinant, comprenant un fragment d'ADN ou un vecteur selon l'une quelconque des revendications 1 à 9.

11. Microorganisme selon la revendication 10, **caractérisé en ce que** sa capacité de catabolisme de la L-carnitine est entièrement ou partiellement inhibée.

12. Microorganisme selon l'une quelconque des revendications 10 et 11, **caractérisé en ce que** sa capacité de recombinaison est altérée.

13. Microorganisme selon l'une quelconque des revendications 10 à 12, sélectionné à partir des microorganismes du genre Escherichia, Pseudomonas, Agrobacterium, Rhizobium, Comamonas et Rhizobium/Agrobacterium.

14. Microorganisme Rhizobium/Agrobacterium HK 1349, comprenant le plasmide pVK100q, tel qu'il est enregistré sous le numéro d'enregistrement DSM 8726, le plasmide figure 3, ou le plasmide pAZ101, tel que **caractérisé par** la carte de restriction selon la figure 4, ainsi que des variantes génétiques et des mutants dérivés de ces microorganismes, possédant la capacité de biosynthèse de la L-carnitine.

15. Microorganisme Rhizobium/Agrobacterium HK 1349.4 comprenant le plasmide pVK100q tel que déposé dans Rhizobium/Agrobacterium HK 1349 sous le numéro d'enregistrement DSM 8726, le plasmide pVK1011, tel que **caractérisé par** la carte de restriction selon la figure 3, ou le plasmide pAZ101, tel que **caractérisé par** la carte de restriction selon la figure 4, ainsi que les variantes génétiques et mutants dérivés de ces microorganismes possédant la capacité de biosynthèse de la L-carnitine.

16. Procédé biotechnologique de production de L-carnitine, **caractérisé en ce que** l'on fait fermenter de la crotonobétaïne et/ou de la γ-butyrobétaïne en présence d'une source appropriée de carbone et d'azote à l'aide d'un microorganisme selon l'une quelconque des revendications 10 à 15 et **en ce que** l'on isole la L-carnitine.
